# FASCICULE DE BREVET EUROPEEN

(11) **EP 1 294 302 B1**
(45) Date de publication et mention de la délivrance du brevet: **06.12.2006**
(21) Numéro de dépôt: 01949569.6
(22) Date de dépôt: 28.06.2001
(51) Int. Cl.: A61B 19/08, A61F 7/00, A47G 9/02

(54) **DISPOSITIF DE DRAPAGE CHIRURGICAL ET DE LUTTE CONTRE LA THERMOLYSE PEROPERATOIRE**
CHIRURGISCHES ABDECKTUCH ZUR BEKÄMPFUNG VON DER INTRAOPERATIVEN THERMOLYSE
SURGICAL DRAPING DEVICE AND ELIMINATION OF PEROPERATIONAL THERMOLYSIS RISKS

(30) Priorité: 29.06.2000 FR 0008742
(43) Date de publication de la demande: 26.03.2003
(73) Titulaire: Carjuzaa, Alain Pierre Xavier, 50120 Equeudreville (FR)
(72) Inventeur: Carjuzaa, Alain Pierre Xavier, 50120 Equeudreville (FR)
(74) Mandataire: Burtin, Jean-François
(86) Numéro de dépôt international: PCT/FR2001/002069
(87) Numéro de publication internationale: WO 2002/000132

(56) Documents cités:
- US-A- 5 443 488
- US-A- 5 728 145
- US-A- 5 755 755
- US-A- 5 800 483
- US-A- 5 941 907
- US-A- 5 964 792

## Description

L'invention concerne un dispositif destiné à éliminer la thermolyse peropératoire des patients subissant une intervention chirurgicale sous anesthésie.

L'hypothermie peropératoire est, de plus en plus, considérée comme une complication sérieuse de l'anesthésie, justifiant la mise en oeuvre de moyens de surveillance et de prévention adaptés à l'importance des risques de perte de chaleur, qui sont principalement fonction de la température ambiante, de la profondeur de l'anesthésie, ainsi que de la nature et de la durée de l'acte chirurgical. En effet, cette hypothermie est source d'inconfort, de retard de réveil ou d'ischémie coronarienne ou cérébrale.

En dehors du champ opératoire, la principale source de déperdition thermique est la peau. Dans ce cas, l'évolution de la température centrale du patient dépend, pour l'essentiel, de la température ambiante et de la protection de celui-ci contre la thermolyse.

Lorsque la température ambiante est supérieure à 21°C, une isolation thermique cutanée suffit à prévenir l'hypothermie, si le champ opératoire est limité et ne comporte pas d'ouverture du thorax ou de l'abdomen. Le confort des équipes chirurgicales, qui impose souvent des températures ambiantes plus basses, de 18 à 19°C, les interventions de longue durée, les terrains à risque (personnes âgées, enfants, coronariens...) impliquent des mesures actives de réchauffement du patient. en vue d'éviter l'hypothermie.

Lorsque l'étendue du champ opératoire le permet, la protection de la quasi-totalité du revêtement cutané peut constituer une mesure efficace. Dans un environnement à 20°C, la perte de chaleur passe de 100 W à 70 W, lorsque le sujet anesthésié est totalement couvert et, ce, quel que soit le matériau utilisé, tel que, par exemple, une simple couverture de coton.

Toutefois, pour des interventions plus lourdes, à champ opératoire étendu, il est nécessaire de recourir à des moyens de réchauffement actifs, dont les plus efficaces utilisent la peau comme surface d'échange : la convection d'air chaud ayant été jugée jusqu'alors la piste la plus intéressante.

Les moyens de lutte les plus couramment utilisés à l'heure actuelle pour lutter contre la thermolyse peropératoire chez les patients subissant une intervention chirurgicale sous anesthésié sont constitués, soit de couvertures de coton peu performantes, soit d'une couverture chauffante par air chaud pulsé, non stérile, utilisée et contrôlée par l'anesthésiologiste-réanimateur, mise en place après le drapage chirurgical. On pourra notamment se reporter aux documents US-A-5941907 (AUGUSTINE SCOTT D) et US-A-5800483 (VOUGHT KIMBER L) qui décrivent des drapages chirurgicaux du genre.

L'association du drapage chirurgical sur le site opératoire et de la couverture chauffante par air pulsé sur l'hémicorps disponible est imparfaite, peu pratique, ne compense que partiellement la thermolyse peropératoire et est source de gêne et d'encombrement.

Il a été proposé par US-A-5941907 de rendre solidaires une couverture transparente permettant de visualiser le visage du patient, avec la couverture chauffante de manière à former un ensemble monobloc. Cependant, si ce drapage tend à faciliter l'intervention du personnel médical quant à mise en oeuvre, il reste insatisfaisant au regard de la lutte contre la thermolyse peropératoire en raison de sa structure même et des modalités de fixation du drapage sur le lit en recouvrement distant du patient, à la manière habituelle dans le domaine.

La présente invention a pour but de remédier à ces inconvénients. Cette invention, telle qu'elle est caractérisée, résout le problème consistant à créer une couverture stérile chauffante, à usage unique, incluse dans la trousse de drapage chirurgical, qui puisse être installée en première approche par le chirurgien, par recouvrement du patient avec une seule pièce, présentant des ouvertures adaptées aux divers sites d'intervention, préalablement réalisées.

Le dispositif de l'invention, est un dispositif de drapage chirurgical et de lutte contre la thermolyse peropératoire des patients subissant une intervention chirurgicale sous anesthésie, comprenant une double paroi contenant un moyen de chauffage, et comprenant un champ de visualisation de la tête du patient et au moins une ouverture adaptée au site d'intervention.

Selon l'invention, ce dispositif de drapage se caractérise principalement en ce qu'il est constitué d'une double paroi contenant le moyen de chauffage, réalisée dans une matière souple non tissée stérile, qui comporte au moins l'ouverture adaptée au site d'intervention chirurgical, ainsi que le champ transparent de visualisation de la tête du patient, et un moyen de contrôle de la température obtenue dans la double paroi de la couverture. Ce dispositif comporte un moyen permettant sa fixation au corps du patient.

Selon un mode de réalisation préférentiel du moyen de fixation du dispositif au corps du patient, les bords jointifs du ou des ouvertures comportent, sur leur face interne, une bande adhésive hypoallergénique, destinée à la fixation par collage directement sur le corps du patient.

Ce dispositif est réalisé en différentes tailles (adulte, enfant, nourisson).

Le moyen de chauffage est préférentiellement de l'air chaud fourni par un générateur à débit et température réglables, muni d'un filtre anti-poussière, auquel un manchon d'insufflation dans la double paroi du dispositif peut être directement et facilement raccordé ; la circulation de l'air chaud s'effectuant dans un labyrinthe constitué de chicanes obtenues par soudage linéaire de la face interne des côtés de la double paroi.

Selon une première variante de réalisation du moyen de chauffage, celui-ci est constitué de résistances électriques.

Selon une deuxième variante de réalisation du moyen de chauffage, celui-ci utilise une combinaison chimique exothermique, à développement lent, déclenchée par la mise en contact volontaire de produits chimiques appropriés, préalablement introduits dans la double paroi.

Les avantages obtenus, grâce à cette invention, consistent principalement en ce que la couverture d'approche dont il s'agit est stérile et à usage unique, et en ce qu'elle est intégrée à la trousse de drapage chirurgical adaptée à l'intervention. Cette couverture possède une grande résistance au déchirement et offre une étanchéité parfaite ; elle recouvre le patient d'une seule pièce en dehors de la zone opératoire, et son épaisseur est telle qu'elle ne gêne nullement le chirurgien lors de l'intervention. Elle possède au moins une ouverture adaptée aux divers sites d'intervention (membres supérieurs. inférieurs, thorax. abdomen, périnée, tête, cou. etc.) ; cette ou ces ouvertures comportant, sur la face interne de leur bord, une bande adhésive hypoallergénique, destinée à leur fixation efficace sur le corps du patient. Dans sa version à chauffage par air chaud pulsé, elle présente un nombre suffisant d'orifices d'entrée d'air chaud et est adaptée à la taille du patient. Elle peut être munie d'une sonde thermique, permettant la surveillance permanente de la température de l'air chaud pulsé sur les moniteur d'anesthésie pour une meilleure sécurité du patient. Le champ transparent qu'elle comporte au niveau de la tête du patient permet la surveillance, par l'anesthésiste, des réactions de celui-ci. Son pliage permet au chirurgien une installation aisée sur le patient en première approche, après badigeonnage par antiseptique du champ opératoire.

Le manchon d'insufflation d'air chaud fait partie intégrante de cette couverture chauffante stérile, intégrée à la trousse de drapage chirurgical, et s'adapte directement sur le générateur d'air chaud pulsé ; il est d'une longueur suffisante pour que le chirurgien ou son aide opératoire puisse le présenter à l'anesthésiste sans commettre de faute d'aseptie, son diamètre correspondant à celui de l'orifice de sortie d'air chaud du générateur. Ce générateur d'air chaud, de capacité suffisante pour la production d'air chaud pulsé, est réglable en débit et en intensité ; il comporte un filtre anti-poussière efficace et est d'encombrement minimum, afin de permettre son adaptation sur les supports des appareils d'anesthésie ; il est conforme aux normes de sécurité.

D'autres caractéristiques et avantages apparaîtront dans la description qui va suivre de couvertures de drapage chauffantes par air chaud, réalisées selon l'invention, données à titre d'exemple non limitatif, au regard des dessins annexés représentant :
- figure 1, une vue de dessus d'une couverture pour chirurgie abdominale adulte,
- figure 2, une vue de dessus d'une couverture pour chirurgie thoracique gauche, en position décubitus latéral droit du patient,
- figure 3, une vue de dessus d'une couverture pour chirurgie du membre inférieur gauche, en position décubitus latéral droit d'un patient adulte,
- figure 4, une vue de dessus d'une couverture pour chirurgie du membre inférieur droit (genou),
- figure 5. une vue de dessus d'une couverture pour chirurgie du rachis cervical (A), dorsal (B), lombaire (C), avec position agenouillée, en décubitus dorsal du patient,
- figure 6, une vue partielle, en coupe selon F, de la couverture au niveau de l'ouverture d'intervention représentée sur la figure 1.

Les figures représentent des couvertures chauffantes de drapage, 1O, 20, 30,40, 50 reliées à un générateur d'air pulsé par un manchon 11, 21, 31, 41, 51 solidaire des couvertures 10, 20, 30, 40, 50, qui comportent, respectivement, une ouverture d'intervention 120, 220, 320, 420 et 520, située au niveau du site d'intervention correspondant, dont la face interne des bords 121 , 221, 321, 421 et 521 est munie d'une bande adhésive hypoallergénique 122, 222, 322, un champ transparent 13, 23, 33, 43, 53 permettant la visualisation du visage du patient, et une sonde thermique 1 d'indication de la température de l'air dans la double paroi 15, 25, 35, 45, 55 des couvertures 10, 20, 30, 40, 50, un manchon de sortie d'air 16, 26, 36, 46, 56.

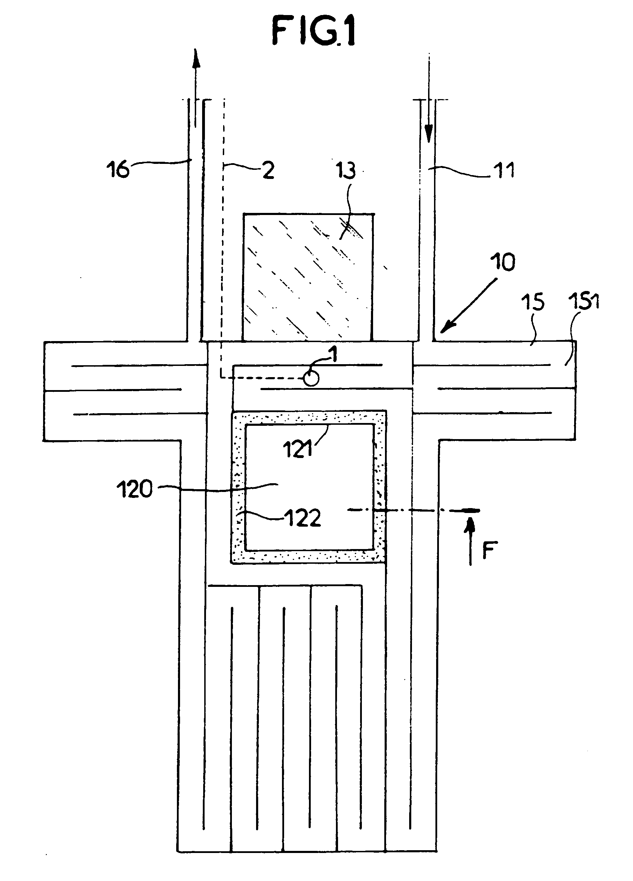

En examinant maintenant plus en détail la figure 1, on remarque que la couverture 10 recouvre la totalité du corps du patient, tout en permettant la surveillance du visage de celui-ci et en dégageant le site d'intervention, sans risque de glissement au cours de l'intervention et, par conséquent, de gêne pour le chirurgien, puisque les bords 121 de l'ouverture d'intervention 120 sont reliés au corps du patient par collage, par l'intermédiaire de la bande adhésive 122 sur la peau du patient, préalablement au raccordement du manchon d'arrivée d'air 11 au générateur d'air chaud pulsé et du manchon de sortie d'air 16 aux moniteurs de contrôle (non représentés), avec recyclage éventuel à l'aspiration du générateur, après épuration par une batterie de filtres (non représentée).

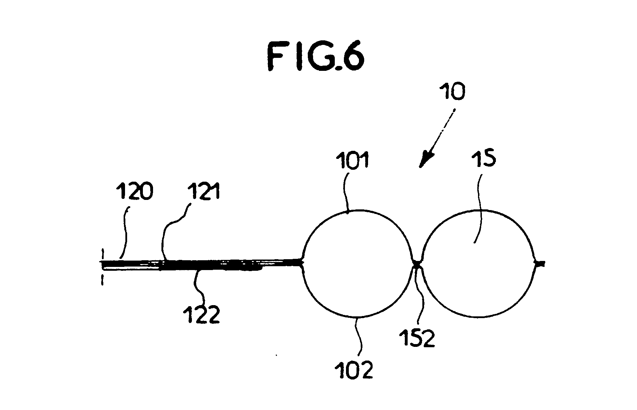

Comme représenté à la figure 6, l'air chaud, pulsé dans la double paroi 15 de la couverture 10, chemine dans celle-ci par des chicanes 151, qui assurent une répartition régulière de la température sur toute la surface de la couverture 10, jusqu'à la sortie de l'air par le manchon 16. Un contrôle à distance, par l'anesthésiste, de la température de l'air à l'intérieur de la couverture est prévu par l'intermédiaire d'une sonde 1, reliée, par un câble 2, à un afficheur (non représenté).
Dans cet exemple, correspondant à une intervention chirurgicale abdominale, la couverture 10 recouvre tout le corps du patient qui est ainsi maintenu à bonne température, quelle que soit la durée de l'intervention.

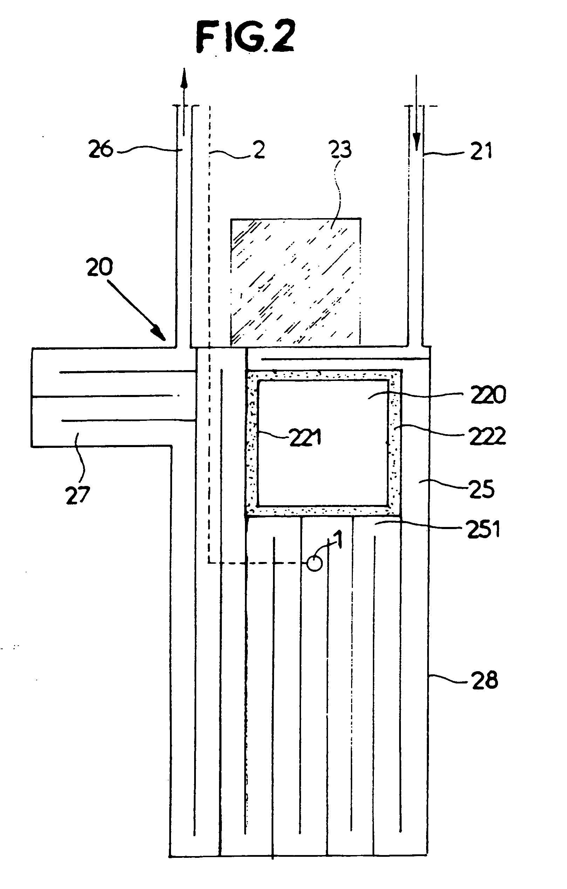

En se rapportant successivement aux figures 2 à 5 :
- Sur la figure 2, correspondant à une intervention chirurgicale thoracique gauche, on remarque que la partie de la couverture 20, qui aurait dû normalement couvrir le bras gauche du patient, a été supprimée, compte-tenu de la position décubitus latéral droit du patient; les autres parties 23, 27 et 28 de la couverture 20 ayant été conservées, avec réalisation à double paroi 25 à chicanes 251, permettant de canaliser l'air chaud du manchon d'arrivée 21 au manchon de sortie 26.
   Comme dans l'exemple précédent, avec contrôle de la température par une sonde 1 avec liaison par câble 2 à un afficheur (non représenté), l'ouverture d'intervention 220 à bords 221 munis de bandes adhésives 222 permet la fixation de ceux-ci au corps du patient, le visage de celui-ci pouvant être surveillé en permanence à travers le champ transparent 23.

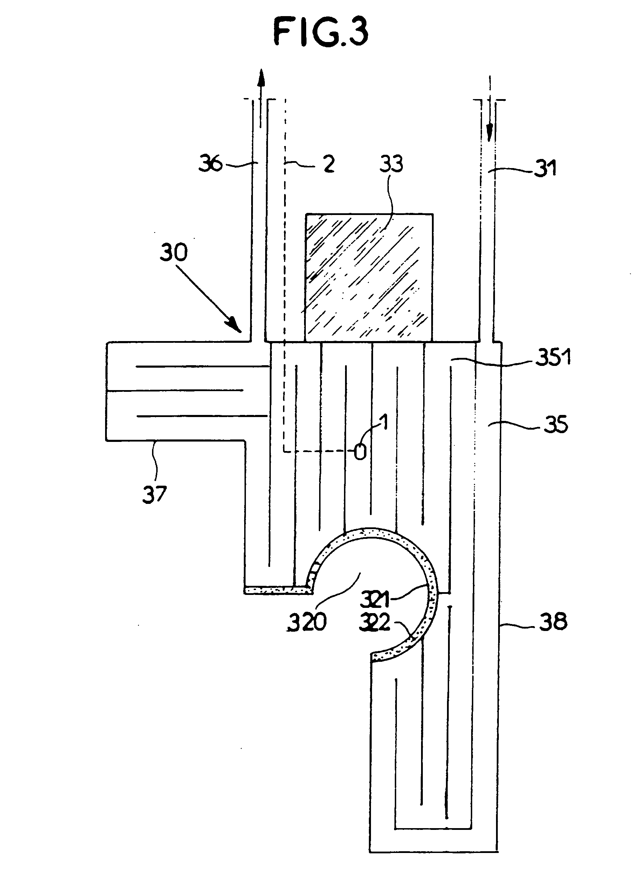

- Sur la figure 3. correspondant à une intervention chirurgicale sur le membre inférieur gauche d'un patient, on remarque que la partie inférieure gauche du bas 38 de la couverture 30, qui aurait dû normalement couvrir la jambe droite du patient, a été supprimée, ainsi que celle qui aurait dû normalement couvrir le bras gauche, compte-tenu de la position décubitus latéral droit du patient, comme dans l'exemple précédent, les autres parties 33 et 37 ayant été conservées, avec réalisation à double paroi 35 à chicanes 351, destinées à canaliser l'air chaud du manchon d'arrivée 31 au manchon de sortie 36, avec contrôle de la température par sonde 1 et câble 2, comme dans les deux exemples précédents. L'ouverture d'intervention 320 à bords 321 est munie de bandes adhésives 322, permettant la fixation de ceux-ci au corps du patient, avec surveillance du visage de celui-ci à travers le champ transparent 33.

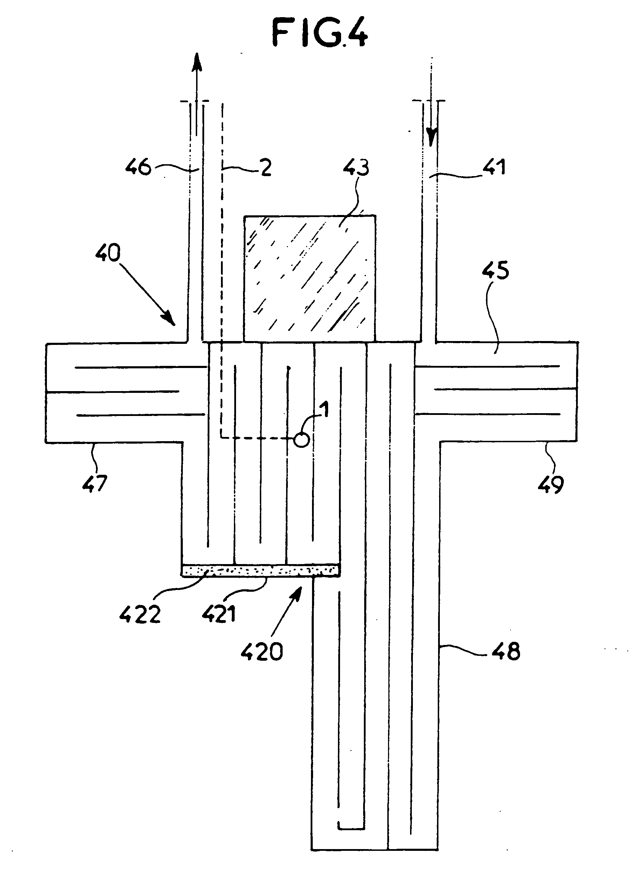

- Sur la figure 4, correspondant à une intervention chirurgicale sur le membre inférieur droit d'un patient (genou par exemple), on remarque que la partie inférieure gauche du bas 48 de la couverture 40, qui aurait du normalement couvrir la jambe droite du patient, a été supprimée, alors que les deux parties 47 et 49, destinées à couvrir les bras de celui-ci, ont été conservées, contrairement à l'exemple précédent. Par contre, dans ce cas, l'ouverture d'intervention 420 est délimitée vers le haut par un bord droit 421, dont la face interne a été munie d'une bande adhésive 422 ; les autres éléments, tels que les manchons d'arrivée et de sortie d'air 41,46, sont inchangés quant à leur position et leur constitution. Il en est de même pour la sonde thermique 1 et son câble de liaison 2, et pour le champ transparent 43.

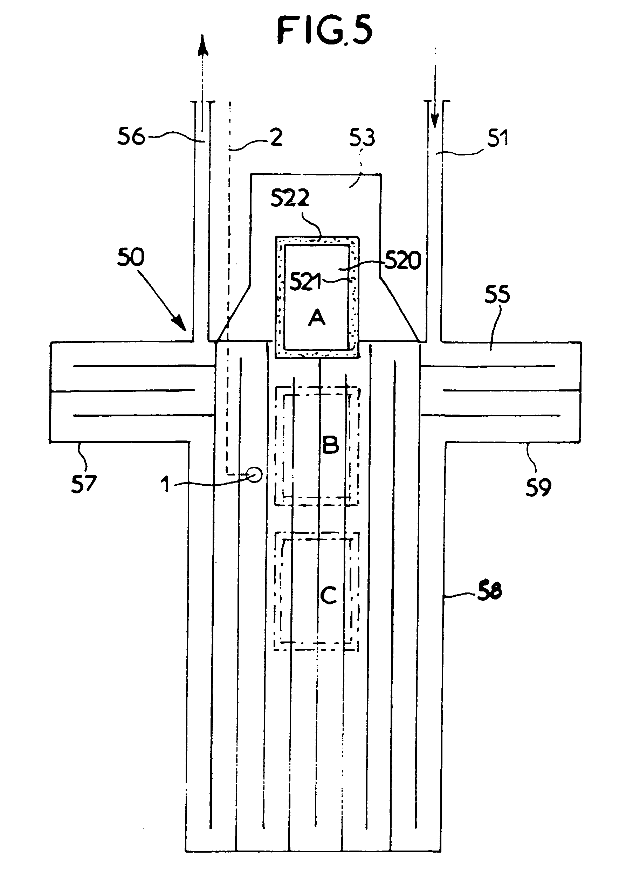

- Sur la figure 5, correspondant à une intervention chirurgicale rachidienne, on remarque que la couverture 50 dont il s'agit se différencie principalement de celle représentée à la figure 1 par un élargissement de la base du champ transparent 53 et par la réalisation, dans celui-ci, d'une ouverture d'intervention sur le rachis cervical, le patient étant en position agenouillée en décubitus dorsal ; cette ouverture 520, correspondant dans ce cas d'intervention à la position (A), comporte, sur la face interne de ses bords 521, une bande adhésive 522, utilisable comme dans tous les exemples précédents. Le bas 58 de la couverture 50 et ses extensions latérales 57 et 59, les manchons 51 et 56 d'arrivée et de sortie d'air, ainsi que la sonde thermique 1 à câble de liaison 2, sont, dans ce mode de réalisation, identiques à ceux réalisés dans la couverture 10 représentée à la figure 1, mais l'ouverture d'intervention 520 peut prendre trois positions différentes selon le site d'intervention rachidien: position (A) pour le rachis cervical, (B) pour le rachis dorsal et (C) pour le rachis lombaire. Ces ouvertures étant, bien évidemment, réalisées de construction par le fabricant de la couverture, selon le site d'intervention rachidien.

Sur la figure 6, correspondant à une vue partielle. en coupe transversale selon F indiquée sur la figure 1, de la couverture 10, au niveau de l'ouverture d'intervention 120, on remarque que les chicanes 151 de circulation d'air chaud sont obtenues par soudage ou collage linéaire, selon une ligne 152 des deux côtés en non tissé 101 et 102 de la double paroi 15, en ménageant, de façon alternée une ouverture de passage d'air pour obtenir des chicanes 151, afin que, comme cela apparaît bien sur toutes les figures, l'air chaud puisse, au cours de son passage dans le labyrinthe ainsi réalisé, maintenir à bonne température toute la face interne de la couverture, avec visualisation de cette température à l'aide de la sonde thermique 1 et de l'afficheur (non représenté) auquel elle est reliée par le câble 2.

L'invention ne se limite pas, nous le rappelons, à la couverture de drapage chauffante par air chaud, dont plusieurs modes de réalisation ont été décrits et représentés à titre d'exemple, car d'autres moyens de chauffage qu'une circulation d'air chaud peuvent être en effet envisagés, tels que le chauffage par résistance électrique, comme cela se pratique pour des couvertures chauffantes domestiques, ou en utilisant une réaction chimique exothermique, dont certaines permettent d'obtenir une température douce d'assez longue durée et sont actuellement commercialisées dans divers domaines.

## Revendications

1. Dispositif de drapage chirurgical et de lutte contre ,la thermolyse peropératoire des patients subissant une intervention chirurgicale sous anesthésie, comprenant une double paroi **(15)** contenant un moyen de chauffage, et comprenant au moins une ouverture **(120)** adaptée au site d'intervention, ainsi qu'un champ transparent **(13)** de visualisation de la tête du patient **caractérisé en ce qu'**il est constitué d'une double paroi, réalisée dans une matière souple non tissée, contenant le moyen de chauffage et comportant au moins l'ouverture **(120)** adaptée au site d'intervention, ainsi que le champ transparent **(13)** de visualisation de la tête du patient, et un moyen de visualisation (1) de la température de l'air dans la double paroi **(15),** pour recouvrir la totalité du corps du patient avec une seule pièce en dehors de la zone opératoire tout en permettant la surveillance du visage de celui-ci.

2. Dispositif selon la revendication 1, **caractérisé en ce que** les bords **(121)** de l'ouverture d'intervention **(120)** sont munis, sur leur face interne, d'une bande adhésive hypoallergénique **(122),** destinée au maintien efficace du dit dispositif sur le corps du patient, par collage sur la peau de celui-ci.

3. Dispositif selon la revendication 1 ou 2, **caractérisé en ce que** le moyen de chauffage est une circulation d'air chaud dans la double paroi **(15).**

4. Dispositif selon là revendication 3, **caractérisé en ce que** la circulation d'air chaud s'effectue dans un labyrinthe constitué de chicanes **(151)** obtenues par soudage linéaire de la face interne des côtés **(101, 102)** de la double paroi **(15).**

5. Dispositif selon la revendication 3 ou 4, **caractérisé en ce que** l'air chaud est fourni par un générateur, réglable en débit et en température, muni d'un filtre anti-poussière, auquel un manchon **(11)** d'arrivée d'air peut être directement raccordé.

6. Dispositif selon l'une des revendications 1 à 5, **caractérisé en ce que** le moyen de chauffage est constitué de résistances électriques.

7. Dispositif selon l'une des revendications 1 à 5, **caractérisé en ce que** le moyen de chauffage est une combinaison chimique exothermique, à développement lent, déclenchée par la mise en contact volontaire de produits chimiques appropriés, préalablement introduits dans la double paroi **(15).**

8. Dispositif selon l'une des revendications 1 à 7, **caractérisé en ce qu'**il est réalisé en différentes tailles.

## Claims

1. Surgical draping device against preoperative thermolysis of the patients undertaking an operation under anesthesia, comprising a double wall (15) containing a heating device, and comprising at least an opening (120) adapted to the operating area as well as a transparent field of view (13) of the patient's head, **characterized in that** it is constituted of a double wall, made of a smooth nonwoven material, containing the heating device and comprising at least the opening (120) adapted to the operation site as well as the transparent field of view (13) of the patient's head, and a display (1) of air temperature in the double wall (15), to cover the whole of the patient's body with a single piece except from the operating area, while allowing the survey of the patient's face.

2. Device according to claim 1, **characterized in that** the rims (121) of the operation opening (120) are equipped on their internal face of a hypoallergenic adhesive tape (122), designed for the effective fixing of the said device on the patient's body by gluing on his skin.

3. Device according to claim 1 or claim 2, **characterized in that** the heating device is a hot air circulation in the double wall (15).

4. Device according to claim 3, **characterized in that** the hot air circulation occurs in a maze constituted of baffles (151) obtained by linear cementing of the internal face of the sides (101, 102) of the double wall (15).

5. Device according to claim 3 or claim 4, **characterized in that** the hot air is supplied by a generator whose flow and temperature are adjustable, equipped with a dust filter, at which an air sleeve (11) may be directly attached.

6. Device according to one of the claims 1 to 5, **characterized in that** the heating device is constituted by electric resistances.

7. Device according to one of the claims 1 to 5, **characterized in that** the heating device is an exothermic slow-development chemical combination, started by voluntarily setting into contact of appropriated chemical compounds, introduced beforehand into the double wall.

8. Device according to one of the claims 1 to 7, **characterized in that** it is realized in different sizes.

## Patentansprüche

1. Vorrichtung zur Chirurgische Einhüllung sowie Bekämpfung der nach Durchoperationsthermolyse von Patienten die eine Chirurgische Eingriff unter Anästhesie erleiden, die eine Heizungsmittel enthält und mindestens eine Öffnung (120), die dem Ort des Eingriffs einpaßt wird und auch ein durchsichtige Beruch (13) umfasst das Aussehen des Kopfs des Patientes erlaubt, **dadurch gegenzeichnet** ist, daß sie aus einem Doppelwand besteht, der mit einem weichen Vliesmaterial realisiert wird, der der Heizungsmittel enthält und die Öffnung (120) für dem Ort des Eingriffs einpassiert, wie auch der durchsichtige Beruch (13) für das Aussehen des Kopfs des Patientes und ein Sichtbarmachungsmittel (1) für die Temperatur des Luftes in dem Doppelwand (15) einschließt, um die Ganzheit des Körpers des Patientes mit eine einzige Tuch zu verhüllen außerhalb des Operationsbereich wenn es auch die Überwachung des Aussehen dieses erlaubt.

2. Vorrichtung nach Anspruch 1, **dadurch gegenzeichnet** ist, daß die Rände (121) der Öffnung des Eingriffs (120) an ihrer innere Seite mit einem hypoallergenischen Klebendenstreife (122) versehen werden, die für eine Wirksame Haltung dieser Vorrichtung an dem Körper des Patientes durch Kleben auf dem Hart dieses bestimmt ist.

3. Vorrichtung nach Anspruch 1 oder 2, **dadurch gegenzeichnet** ist, daß der Heizungsmittel aus einem Kreislauf von warmen Luft in dem Doppelwand (15) besteht.

4. Vorrichtung nach Anspruch 3, **dadurch gegenzeichnet** ist, daß der Kreislauf von warmen Luft in einem aus Hindermitse (151) bestehende Labyrinthe, die durch linearen Schweißen der inneren Lagen der Seite (101, 102) des Doppelwandes (15) hergestellt wird, sich vollzieht.

5. Vorrichtung nach Anspruch 3 oder 4, **dadurch gegenzeichnet** ist, daß der warme Luft durch eine Generator, der für Durchfluß und für Temperatur verstellbar ist, mit einem Gegenstaubfilter versehen ist, zu dem eine Muffe (11) für die Luftzufuhr unmittelbar gekoppelt werden kann.

6. Vorrichtung nach einer der Ansprüche 1 bis 5, **dadurch gegenzeichnet** ist, daß der Heizungsmittel aus elektrischen Widershände besteht.

7. Vorrichtung nach einer der Ansprüche 1 bis 5, **dadurch gegenzeichnet** ist, daß der Heizungsmittel aus einer chemischen exothermischen Vermischung, die eine langsame Entwicklung hat, besteht, die mit eigenwilligen in Beruhrungsbringung von eigneten vorher in dem Doppelwand (15) hinein gesteckte, ausgelost wird.

8. Vorrichtung nach einer der Ansprüche 1 bis 7, **dadurch gegenzeichnet** ist, daß sie in verschiedenen Größe durchgeführt wird.
